# EUROPEAN PATENT SPECIFICATION

(11) **EP 1 455 839 B1**
(45) Date of publication and mention of the grant of the patent: **23.01.2013**
(21) Application number: 99951885.5
(22) Date of filing: 08.10.1999
(51) Int. Cl.: A61K 47/48, A61K 47/34, C08G 65/329

(54) **HETEROBIFUNCTIONAL POLY(ETHYLENE GLYCOL) DERIVATIVES AND METHODS FOR THEIR PREPARATION**
HETEROBIFUNKTIONELLE POLYETHYLENGLYCOL DERIVATE UND VERFAHREN ZU IHRER HERSTELLUNG
DERIVES HETEROBIFONCTIONNELS D'ETHYLENE-GLYCOL ET POLYETHYLENE-GLYCOLE ET PROCEDES D'ELABORATION

(43) Date of publication of application: 15.09.2004
(73) Proprietor: Nektar Therapeutics, San Carlos, CA 94070 (US)
(72) Inventor: BENTLEY, Michael, David, Huntsville, AL 35801 (US); HARRIS, J., Milton, Huntsville, AL 35801 (US); KOZLOWSKI, Antoni, Huntsville, AL 35816 (US)
(74) Representative: Vossius & Partner
(86) International application number: PCT/US1999/023536
(87) International publication number: WO 2001/026692

(56) References cited:
- EP-A- 0 867 190
- WO-A-97/03106
- WO-A-99/14259
- WO-A-99/34833
- WO-A-99/45964
- ZHAO X ET AL: "NOVEL DEGRADABLE POLY(ETHYLENE GLYCOL) HYDROGELS FOR CONTROLLED RELEASE OF PROTEIN" JOURNAL OF PHARMACEUTICAL SCIENCES,US,AMERICAN PHARMACEUTICAL ASSOCIATION. WASHINGTON, vol. 87, no. 11, 1 November 1998 (1998-11-01), pages 1450-1458, XP000783392 ISSN: 0022-3549

## Description

### Field of the Invention

The invention relates to heterobifunctional poly(ethylene glycol) derivatives and methods for their preparation.

### BACKGROUND OF THE INVENTION

Covalent attachment of the hydrophilic polymer poly(ethylene glycol), abbreviated (PEG), also known as poly(ethylene oxide), abbreviated (PEO), to molecules and surfaces has important applications in biotechnology and medicine. In its most common form, PEG is a linear polymer having hydroxyl groups at each terminus:

HO-CH₁-CH₂O(CH₂CH₂O)ₙCH₂CH₂-OH

This formula can be represented in brief as HO-PEG-OH, where it is understood that -PEG- represents the polymer backbone without the terminal groups:

-PEG- equals -CH₂CH₂O(CH₂CH₂O)ₙCH₂CH₂-

PEG is commonly used as methoxy-PEG-OH, or mPEG in brief, in which one terminus is the relatively inert methoxy group, while the other terminus is a hydroxyl group that is subject to ready chemical modification.

CH₃O-(CH₂CH₂O)ₙ-CH₂CH₂-OH

It is understood by those skilled in the art that the term poly(ethylene glycol) or PEG represents or includes all the above forms and still others.

The copolymers of ethylene oxide and propylene oxide are closely related to PEG in their chemistry, and they can be substituted for PEG in many of its applications.

HO-CH₂CHRO(CH₂CHRO)ₙCH₂CHR-OH

R = H and CH₃

PEG is a useful polymer having the property of water solubility as well as solubility in many organic solvents. PEG is also non-toxic and non-immunogenic. When PEG is chemically attached to a water insoluble compound, the resulting conjugate generally is water soluble as well as soluble in many organic solvents. When the molecule to which PEG is attached is biologically active, such as a drug, this activity is commonly retained after attachment of PEG and the conjugate may display altered pharmacokinetics. For example, Bentley et al. in Polymer Preprints, 38(1), 584 (1997) demonstrated that the water insoluble antemisinin becomes water soluble and exhibits increased antimalarial activity when coupled to PEG. Davis et al., in U.S. Patent No. 4,179,337 have shown that proteins coupled to PEG have enhanced blood circulation lifetime because of reduced kidney clearance and reduced immunogenicity. The lack of toxicity of PEG and its rapid clearance from the body are advantageous for pharmaceutical applications.

As applications of PEG chemistry have become more sophisticated, there has been an increasing need for heterobifunctional PEGs, that is PEGs bearing dissimilar terminal groups:

X-PEG-Y

where X and Y are different groups. PEGs having backbone ester groups and terminal groups, X and Y:

X-PEG-CO₂-PEG-Y

can be considered to be heterobifunctional even if X and Y are the same, since each PEG unit within the backbone is substituted unsymmetrically.

Such heterobifunctional PEGs bearing appropriate functional groups may be used to link the PEGs to surfaces or other polymers, such as polysaccharides or proteins, with the other terminus attached, for example, to a drug, a liposome, another protein, or a biosensor. If one terminus is bound to a polymer, and the other terminus is bonded to an appropriate functional group, cross-linking to form a useful hydrogel can occur. WO 1999/034833 A1 discloses degradable heterobifunctional poly (ethylene glycol) acrylates and gels and conjugates derived therefrom.

Utilizing existing methods, however, heterobifunctional PEGs are often difficult or impossible to prepare in high purity. For example, one could conduct the below reaction, using molar equivalents of each reagent with the goal of preparing the heterobifunctional PEG acetal product shown:

HO-PEG-OH + ClCH₂CH(OC₂H₅)₂ + NaOH → → HO-PEG-OCH₂CH(OC₂H₃)₂+NaCl+H₂0

In practice, however, some of the disubstituted PEG diethyl acetal, (C₂H₅O)₂CH₂O-PEO-OCH₂CH(OC₂H₅)₂ is also inevitably formed and some unreacted PEG would also remain. Tedious chromatography would be required to separate this mixture.

The chromatographic approach has been used by Zalipsky (Bioconjugate Chemistry, 4: 296-299, 1993) to purify the following heterobifunctional PEG derivative:

HO-PEG-CONHCH₂CO₂H

from a reaction product mixture also containing unreacted PEG and the disubstituted carboxylic acid derivative.

In certain applications, it is essential that minimum HO-PEG-OH be present in monoalkyl PEGs used to prepare monofunctional activated PEGs, since the presence of HO-PEG-OH would lead to doubly activated PEG derivatives which would result in crosslinked products or have other undesirable effects. In fact, HO-PEG-OH is a common contaminant in monoalkyl PEGs. The chromatographic approach has been disclosed in U.S. Patent No. 5,298,410 to separate CH₃O-PEG-OH from HO-PEG-OH by forming the trityl (Ph₃C-derivatives), separating the derivatives chromatographically, and removing the trityl group from CH₃O-PEG-OCPh₃. A recent patent application, Suzawa, *et al*. (WO96/35451) disclosed benzyl PEG (C₆H₃-CH₂-OPEG-OH) as an intermediate in preparing a heterobifunctional PEG bearing a group at one terminus having affinity for a target cell and having a toxin at the other terminus. The benzyl PEG, however, was prepared by benzylation of PEG, followed by laborious extensive gradient chromatography to separate benzyl PEG from dibenzyl PEG and unreacted PEG. The procedure was done on a small scale with a yield of only 7.8%. The method thus has little value for useful commercial production.

A second strategy, the polymerization approach, for preparing heterobifunctional PEGs involves anionic polymerization of ethylene oxide onto an anion, X⁻, which ultimately becomes the end-group of the polymer: 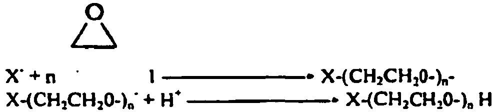 This method has been used by Yokoyama, et al. (Bioconjugate Chemistry, 3: 275-276, 1992) to prepare a PEG with a hydroxyl group at one terminus and an amino group at the other. Cammas, et al. (Bioconjugate Chemistry, 6: 226-230, 1995) have used this method to prepare PEGs with an amino group on one terminus and a hydroxyl or methoxy group on the other. It has also been used by Nagasaki, et al. (Biocojugate Chemistry, 6: 231-233, 1995) to prepare a PEG having a formyl group at one terminus and a hydroxyl group at the other. This method is generally useful only if X is a suitable and desired group on which to initiate polymerization; frequently this is not the case. Also, successful application of this method requires rigorous exclusion of water to prevent formation of HO-PEG-OH, and this problem becomes more severe as the molecular weight increases. It is also necessary to carefully control the degree of polymerization in order to obtain the desired molecular weight of the PEG derivative. This method is limited by the degradation of many types of drug molecules under the harsh conditions of the polymerization if the ethylene oxide polymerization is conducted directly on the drug molecule. The method is also limited by lack of selectivity if more than one functional group is present on which polymerization can occur.

It would be desirable to provide additional methods for preparing heterobifunctional PEGs that substantially eliminate at least some of the problems and drawbacks of previous methods.

### SUMMARY OF THE INVENTION

Within the scope defined by the annexed claims, this invention provides a method for preparing heterobifunctional poly(ethylene glycol) derivatives via a PEG intermediate bearing a removable group at one terminus. PEG derivatives of the class, W-PEG-OH, where W is a group removable by mild chemical methods, are provided and are first altered by modifying the OH group to a desired group, X followed by removal of W to generate a second hydroxyl group. The latter hydroxyl group may then be further altered to a second functional group Y, thus providing the desired heterobifunctional PEG:

W-PEG-OH W-PEG-X - HO-PEG-X - Y-PEG-X

A preferred removable group is the benzyloxy group (C₆H₅CH₂-O-), although other arylmethyl groups including, but not limited to, 4--methylbenzyl, 3-methylbenzyl. 4-chlorobenzyl, 4-methoxybenzyl, diphenylmethyl, triphenylmethyl, or 1-naphthyimethyl, may be used. Diarylmethyl and triarylmethyl groups will also suffice. Benzyloxy-PEG-OH (BzO-PEG-OH), for example, may be prepared in high purity by polymerization of ethylene oxide onto the benzyloxide ion, BzO⁻. By conducting the reaction under carefully controlled, anhydrous conditions, the heterobifunctional derivative product can be prepared with a minimum amount of HO-PEG-OH. An advantage of benzyl and other arylmethyl groups is that they may be removed from the PEG under relatively mild conditions by catalytic hydrogenolysis or by acid-catalyzed hydrolysis.

BzO-PEG-X + H₂ (*cat*) → C₆H₅-CH₃ + HO-PEG-X

BzO-PEG-X + H₂0 (H⁺) → C₆H₅CH₂0H + HO-PEG-X

In the reactions above, *cat* is a catalyst such as palladium on charcoal.

In one embodiment of the invention, the method is used in conjugating PEG or related polymers to macromolecules such as proteins, lipids, polysaccharides, or other polymers or surfaces. First, the hydroxyl group of the intermediate polymer BzO-PEG-OH is converted to a first reactive functional group. This reactive functional group allows the attachment of the BzO-PEG- to a macromolecule. The benzyl group is then removed by hydrogenolysis or hydrolysis, without chemically affecting the macromolecule, thus making available a new terminal hydroxyl group on the PEG derivative. This new hydroxyl group may be used directly to attach that terminus of the PEG derivative to the same or another macromolecule. Alternatively, the hydroxyl group may be further converted to a second reactive functional group, which is then used to link the PEG derivative to a macromolecule. If the second reactive functional group is linked to another polymer, a cross-linked polymer useful as a hydrogel may be generated. The reaction scheme may be illustrated in a general form as follows:

| | | | | |
|---|---|---|---|---|
| (1) | BzO-PEG-OH | → | BzO-PEG-X | (X=reactive functional group) |
| (2) | BzO-PEG-X | → | BzO-PEG-M₁ | (M₁= a macromolecule, e.g., surface, drug, protein, or polymer.) |
| (3) | BzO-PEG-M₁ + H₂ (Pd/C) | → | BzH + HO-PEG-M₁ | |
| | OR | | | |
| | BzO-PEG-M₁ + H20/H⁺ | → | BzOH + HO-PEG-M₁ | |
| (4) | HO-PEG-M₁ M₂-PEG-M₁ | → | | (M₂= a macromolecule, e.g., surface, drug, protein, or polymer, or a different site on M₁.) |

If desired, the order of the reaction sequence can be manipulated to avoid destruction of a chemical group sensitive to hydrogenation or hydrolysis:

| | | | | |
|---|---|---|---|---|
| (1) | Bz-PEG-OH | → | BzO-PEG-X | |
| (2) | BzO-PEG-X | → | BzO-PEG-M₂ | (M₂=drug, surface, polymer, or other group not sensitive to hydrogenation or hydrolysis.) |
| (3) | BzO-PEG-M₂ + H₂ (Pd/C) | → | BzH (or BzOH) + HO-PEG-M₂ | |
| | OR | | | |
| | BzO-PEG-M₂ + H₂0/H+ | → | BzH (or BzOH) + HO-PEG-M₂ | |
| (4) | HO-PEG-M₂ | → | M₁-PEG-M₂ | (M₁=drug, surface, polymer, or other group sensitive to hydrogenation or hydrolysis.) |

In another embodiment of the invention, a method of inhibiting the reactivity of HO-PEG-OH in a mixture of W-O-PEG-OH and HO-PEG-OH is disclosed. In this approach, alkylation of W-O-PEG-OH containing HO-PEG-OH produces a mixture of W-O-PEG-OR and RO-PEG-OR, where R is an alkyl group.

BzO-PEG-OH + HO-PEG-OH + R-X - BzO-PEG-OR + RO-PEG-OR + HX

X is a leaving group such as mesylate or tosylate.
Catalytic hydrogenation converts the BzO-PEG-OR to RO-PEG-OH.

BzO-PEG-OR + RO-PEG-OR + H₂ (Pd/C) - RO-PEG-OH + RO-PEG-OR + BzH

Thus, a mixture of RO-PEG-OH and RO-PEG-OR is produced.
Unlike HO-PEG-OH, RO-PEG-OR is inert and nonreactive. Thus, the mixture is equivalent to a pure product of RO-PEG-OH in most chemical reactions.

The foregoing and other objects, advantages, and features of the invention, and the manner in which the same are accomplished, will be more readily apparent upon consideration of the following detailed description of the invention.

### DETAILED DESCRIPTION OF THE INVENTION

This invention provides a method for preparing, in high purity and high yield, heterobifunctional derivatives of poly(ethylene glycol) or related polymers. A chromatographic purification step is not necessary in the method. In accordance with the method of the invention, an intermediate polymer having a formula of W-Poly-OH is provided bearing a removable group W at one terminus. The intermediate polymer W-Poly-OH is first altered by modifying the OH group to a first functional group X, followed by the removal of W to generate a second hydroxyl group. The latter hydroxyl group may then be further converted to a second functional group Y, thus providing the desired heterobifunctional derivative:

W-Poly-OH → W-Poly-X → HO-Poly-X → Y-Poly-X

In the discussion below, Poly will often be referred to for convenience as PEG or as poly(ethylene glycol). However, it should be understood that other related polymers as defined in the annexed claims are also suitable for use in the practice of the invention and that the use of the term PEG or poly(ethylene glycol) is intended to be inclusive and not exclusive in this respect.

Poly(ethylene glycol) or PEG is useful in biological applications because it has properties that are highly desirable and is generally approved for biological or biotechnical applications. PEG typically is clear, colorless, odorless, soluble in water, stable to heat, inert to many chemical agents, does not hydrolyze or deteriorate, and is nontoxic. Poly(ethylene glycol) is considered to be biocompatible, which is to say that PEG is capable of coexistence with living tissues or organisms without causing harm. More specifically, PEG is not immunogenic, which is to say that PEG does not tend to produce an immune response in the body. When attached to a moiety having some desirable function in the body, the PEG tends to mask the moiety and can reduce or eliminate any immune response so that an organism can tolerate the presence of the moiety. Accordingly, the heterobifunctional derivative of the invention should be substantially non-toxic and should not tend substantially to produce an immune response or cause clotting or other undesirable effects.

PEG having the formula -CH₂CHO₂-(CH₂CH₂O)ₙ-CH₂CH₂-, where n is from about 8 to about 4000, is one useful polymer in the practice of the invention. Other difunctional, water soluble, nonpeptidic polymers than PEG as defined in the annexed claims are also suitable for the present invention. These other polymers include other poly(alkylene oxides) such as poly(propylene glycol) ("PPG") and the like; and poly(oxyethylated polyols) such as poly(oxyethylated glycerol), poly(oxyethylated sorbitol), and poly(oxyethylated glucose). The polymers can be homopolymers or random or block copolymers and terpolymers based on the monomers of the above polymers, straight chain or branched.

A suitable additional polymer is difunctional poly(acryloylmorpholine) ("PAcM"), and its synthesis and use are described in U.S. Patent Nos. 5,629,384 and 5,631,322.

The terms "group," "functional group," "moiety," "active moiety," and "reactive site," are all somewhat synonymous in the chemical arts and are used in the art and herein to refer to distinct, definable portions or units of a molecule and to units that perform some function or activity and are reactive with other molecules or portions of molecules.

The term "linkage" is used to refer to groups that normally are formed as the result of a chemical reaction and typically are covalent linkages.

It should be understood that by "drug" is meant any substance intended for the diagnosis, cure, mitigation, treatment, or prevention of disease in humans and other animals, or to otherwise enhance physical or mental well being.

The term "macromolecule" is used to mean large molecules including, but not limited to, lipid, polysaccharide, proteins, nucleotide sequences, drugs, polymers, etc. It is often desirable to conjugate the above described polymers to such macromolecules.

In accordance with the invention, the removable group W can be removed from the polymer W-Poly-X by mild chemical reactions. Such chemical reactions can be performed in conditions under which other moieties of the polymer W-Poly-X, particularly the first functional group X, are not undesirably modified. Preferably W has a formula of Ar-C(R₁)(R₂)-O- where Ar represents a moiety selected from the group consisting of phenyl, substituted phenyl, biphenyl, substituted biphenyl, polycyclic aryls, substituted polycyclic aryls, and heterocyclic aryls, where R₁ and R₂ are H, alkyl, or Ar which is defined above. Thus, exemplary examples of the removable group W include, but are not limited to, benzyloxy group (C₆H₅CH₂-O-), and other arylmethyloxy groups including, but not limited to, 4-methylbenzyloxy, 3-methylbenzyloxy, 4-chlorobenzyloxy, 4-methoxybenzyloxy, diphenylmethyloxy, triphenylmethyloxy, and 1-naphthylmethyloxy. The arylmethyloxy groups can be removed from the polymer under relatively mild conditions by catalytic hydrogenolysis or acid-catalyzed hydrolysis.

In accordance with the invention, the intermediate polymer of W-Poly-OH is preferably synthesized, e.g., by polymerization of monomers of the suitable polymers onto the arylmethyloxide ion. For example, Benzyloxy-PEG-OH (BzO-PEG-OH) may be prepared in high purity and high yield by polymerization of ethylene oxide onto the benzyloxide ion BzO-. Preferably, the polymerization reaction is conducted under anhydrous conditions. In accordance with this aspect of the invention, production of HO-PEG-OH is minimized. Extensive gradient chromatography purification is not required, and the yield of BzO-PEG-OH is high. This is in contrast with the prior art method of benzylation of PEG followed by laborious extensive gradient chromatography, which inevitably leads to high cost and low yield rendering the method of little value for commercial production.

In accordance with the invention, the final product of the heterobifunctional derivative of poly(ethylene glycol) or related polymers have a formula of Y-Poly-X. The first functional group X and the second functional group Y are reactive moieties that are capable of reacting with other molecules to which the PEG derivatives are desired to be conjugated to. e.g., macromolecules including, but not limited to, proteins, lipids, polysaccharides, and other polymers. The first functional group X is selected from mesylate; tosylate; tresylate; -O-(CH₂)ₙ-CO₂R₃ where n = 1-6 and R₃ is an alkyl group; -NHR₄ where R₄ is H or alkyl or an amine protecting group such as t-Boc and Fmoc; -O-(CH₂)ₙ-CH(ZR₅)₂ where n is a number of 1-6, Z is O or S, R₅ is H or an alkyl group; aryls, and heterocyclic aryls; and -O-(CH₂)ₙ-CHO.

Examples of the second functional group Y include, but are not limited to, hydroxyl group; mesylate; tosylate; tresylate; -O-(CH₂)ₙ-CO₂H where n = 1-6; -O-(CH₂)ₙ-CO₂R₃ where n = 1-6 and R₃ is an alkyl group; -NHR₄ where R₄ is H or alkyl or an amine protecting group such as t-Boc and Fmoc; -o-(CH₂)ₙ-CH(ZR₅)₂ where n is a number of 1-6, Z is O or S, R₅ is H or an alkyl group; Ar-CH=CH-CH=CH-CO₂- where Ar represents a moiety selected from the group consisting of phenyl, substituted phenyl, biphenyl, substituted biphenyl, polycyclic aryls, substituted polycyclic aryls, and heterocyclic aryls; -O-(CH₂)ₙ-CHO; -O₂CCH₂CH₂CO₂R₆, where R₆ is H or NHS which represents N-succinimidyl; and CH₂=CH-CO₂-. In a poly(ethylene glycol) derivative of the formula Y-Poly-X, the first functional group X and the second functional group Y are different from each other, thus ensuring the polymer to be heterobifunctional.

Preferably, when X is -O-(CH₂)ₙ-CH(ZR₅)₂ where n is a number of 1-6, Z is O or S, and R₅ is H or an alkyl group, Y is preferably -O₂CCH₂CH₂CO₂R₆ where R₆ is H or NHS.

Although the reaction scheme of method of the present invention is demonstrated above in the equations:

W-Poly-OH → W-Poly-X → HO-Poly-X → Y-Poly-X,

it should be understood that there can be more than one chemical reaction steps between any of two products in the equations. For example, several sequential reaction steps may take place to convert the terminal hydroxyl group of W-Poly-OH to the first functional group X. Likewise, several reaction steps may be performed to modify the new hydroxyl group of HO-Poly-X to produce the second functional group Y.

Further, in one embodiment of the invention, before the step of removing the removable group W, the polymer W-Poly-X can be linked to a macromolecule or surface through a linkage formed between the first reactive functional group X and a suitable moiety on the surface of the macromolecule, thus conjugating the W-Poly- portion of the polymer to the macromolecule: W-Poly-M₁ where M₁ is a macromolecule such as protein, peptide, lipid, drug, polysaccharide, or other polymers, or surface of a substance, e.g., microorganism. The removable group W in the conjugate W-Poly-M₁ is thereafter removed by mild chemical reactions such as, e.g., catalytic hydrogenolysis or by acid-catalyzed hydrolysis. The resultant -OH can be reacted directly to, e.g., another macromolecule M₂ such as protein, peptide, lipid, drug, polysaccharide, or other polymers, or surface of a substance, e.g., microorganism to form M₂-Poly-M₁. If conjugation to another macromolecule is not desired, the -OH group can be optionally converted to an inert non-reactive group, e.g., capped by alkylation. Alternatively, the resultant - OH group can be converted to a reactive functional group Y as described above: Y-Poly-M₁. The functional group Y can then be reacted with M₂ to form M₂-Poly-M₁.

| | | | | |
|---|---|---|---|---|
| (1) | W-Poly-OH | → | W-Poly-X | (X=reactive functional group) |
| (2) | W-Poly-X | → | W-Poly-M₁ | M₁= a macromolecule, e.g., surface, drug, protein, or polymer.) |
| (3) | W-Poly-M, + H₂ (Pd/C) | → | WH + HO-Poly-M₁ | |
| | OR | | | |
| | W-Poly-M₁ + H₂O/H⁻ | → | WOH + HO-Poly-M₁ | |
| (4) | HO-Poly-M₁ | → | Y₂-Poly-M₁ | |
| (5) | Y-Poly-M₁ + M₂ | → | M₂-Poly-M₁ | (M₂= a macromolecule, e.g., surface, drug, protein, or polymer, or a different site on M₁.) |

Hydrogels can be produced in this method through crosslinking of multiple different macromolecules by the PEG related polymer Poly. However, it should be understood that, in accordance with this invention, the two functional group X and Y can also be linked to the same macromolecule, wherein the conjugating of PEG related polymers on the macromolecule may form a polymer shell on the macromolecule.

In another embodiment of the invention, the heterobifunctional derivative of PEG or related polymer Y-Poly-X made as described above can be reacted with macromolecules or other substances through the functional groups X and Y and the reactive moieties on the macromolecules or other substances. For example, X and Y can be selected such that two different types of macromolecule or other substance can be linked to X and Y respectively. It is also possible to select X and Y such that they react with the same types of macromolecules.

The following examples are given to illustrate the invention, but should not be considered in limitation of the invention.

| | |
|---|---|
| Example 1. | Synthesis of HO-PEG-NH₃⁺ Cl⁻ |
| Example 2. | Synthesis of HO-PEG -OCH₂CO₂H |
| Example 5. | Synthesis of C₆H₅-CH=CH-CH=CH-CO₂PEG-OCH₂CH(OC₂H₃)₂ |
| Example 9. | Synthesis of NH-0₂CCH₂CH₂COO-PEG-OCH₂CH₂CH(OC₂H₅)₂ |

### EXAMPLE 1

### PREPARATION OF HO-PEG-NH₃⁺Cl⁻

### REACTIONS:

BzO-PEG-OH + MsCl + (CH₃CH₂)₃N → BzO-PEG-OMs + (CH₃CH₂)₃NH⁺Cl⁻

BzO-PEG-OMs + 2NH₃ → BzO-PEG-NH₂ + NH₄⁺OMs⁻

BzO-PEG-NH₂ + H₂O + HCl → HO-PEG-NH₃+Cl⁻ + BzOH

a) Preparation of BzO-PEG-OMs: BzO-PEG-OH (MW=3400, 34 g, 10 mmole) in 150 ml of toluene was azeotropically distilled for 2 hours under nitrogen and the solution was cooled to room temperature. To the solution was added 40 ml of dry methylene chloride and 2.1 ml of dry triethylamine (15 mmole). The solution was cooled in an ice bath and 1.2 ml of dry mesyl chloride (15 mmole) was added dropwise. The solution was stirred at room temperature under nitrogen overnight and the reaction was quenched by adding 2 ml absolute ethanol. The mixture was evaporated under vacuum to remove solvents, primarily those other than toluene, filtered, concentrated again under vacuum, and then precipitated into 100 ml of ethyl ether. The product was collected by filtration and dried *in vacuo*. Yield 34 g (100%). ¹H nmr (DMSO-d₆): δ 3.5 (br m, PEG), 4.31 (t, OCH₂CH₂OMs), \4.49 (s, C₆H₅-CH₂-OPEG-), 7.33 (s+complex mult., C₆H₅-CH₂-OPEG-).
b) Preparation of BzO-PEG-NH₂: BzO-PEG-OMs (25 g, 7.35 mmole) was dissolved in 500 ml of aqueous ammonia solution containing 5 g of ammonium chloride and the solution was stirred at room temperature for 72 hours. The solution was then extracted three times with methylene chloride. The organic phase was dried over sodium sulfate, filtered, condensed under vacuum, and the product precipitated with 100 ml of ethyl ether. The product was collected by filtration and dried *in vacuo* Yield 23 g (92%). ¹H nmr (DMSO-d₆): δ 3.5 (br m, PEG), 2.9 (t, -CH₂NH₂), 4.49 (s, C₆H₅-CH₂-OPEG-), 7.33 (s+complex mult., C₆H₅-CH₂-OPEG-).
c) Preparation of HO-PEG-NH₃⁺Cl⁻: A solution of BzO-PEG-NH₂ (46 g, 14 mmoles) in 200 ml of concentrated HCl (12 M) was stirred at room temperature for 44 h. It was then diluted to 1200 ml with water and NaCl was added to make a 15% solution. The aqueous solution was extracted three times with methylene chloride and the combined extracts were dried over sodium sulfate. The methylene chloride was concentrated under vacuum and the product precipitated by the addition of ether. The product was collected by filtration and dried under vacuum at room temperature. Yield: 42 g (95%). ¹H nmr (DMSO-d₆): δ 2.96 (t, CH₂-N), 3.5 (br m, PEG), 4.6 (br, OH), 7.9 (br, NH₃⁺).

### EXAMPLE 2

### PREPARATION OF HO-PEG-OCH₂CO₂H

### REACTIONS:

BzO-PEG-OH + (CH₃)₃CO⁻K⁺ → BzO-PEG-O⁻K⁺ + (CH₃)₃COH

BzO-PEG-O⁻K⁺ + Br-CH₂CO₂C(CH₃) → → BzO-PEG-O- CH₂CO₂C(CH₃)₃ + KBr

BzO-PEG-O- CH₂CO₂C(CH₃)₃ + H₂O (H⁺) → → HO-PEG-O-CH₂CO₂H + BzOH + (CH₃)₃COH

a) Preparation of BzO-PEG-OCH₂CO₂C(CH₃)₃: BzO-PEG-OH (MW=3400, 40 g, 11.7 mmole) was azeotropically dried with 250 ml toluene under N₂. After two hours, the solution was cooled to room temperature. Potassium tert-butoxide (2.8 g, 23.5 mmole) dissolved in 90 ml tert-butanol and 90 ml toluene was added to the above PEG solution. The mixture was stirred for two hours at room temperature. Tert-butyl bromoacetate (4 ml, 26.3 mmole) was added, and the solution was stirred under N₂ at room temperature overnight. The solution was filtered, condensed under vacuum, and precipitated into 300 ml of ether. The product was collected by filtration and dried under vacuum. ¹H nmr (DMSO-d₆): δ 1.5 (s, t-Bu), 3.51 (m, PEG), 3.98 (s, -OCH₂CO₂-), 4.49 (s, C₆H₅CH₂O-), 7.33 (s + comp. mult., C₆H₅CH₂O-).
b) Preparation of HO-PEG -OCH₂CO₂H: BzO-PEG-OCH₂CO₂C(CH₃)₃ (10 g) was dissolved in 100 ml of hydrochloric acid (37%), and the solution was stirred at room temperature for 48 hours. The solution was diluted with one liter of distilled water and the pH was adjusted to 2 with 1N sodium hydroxide solution. The solution was then extracted three times with methylene chloride. The organic phase was dried over anhydrous sodium sulfate, filtered to remove salt, condensed under vacuum, and precipitated into ether. The product was collected by filtration and dried under vacuum. Yield 8.5 g (85%). ¹H nmr (DMSO-d₆): δ 3.51 (br m. PEG), 4.01 (s, -PEGOCH₂COOH).

### EXAMPLE 5

### PREPARATION OF C₆H₅-CH=CH-CH=CH-CO₂PEG-OCH₂CH(OC₂H₅)₂

### REACTIONS:

BzO-PEG-OH + ClCH₂CH(OC₂H₂) + NaOH → BzO-PEG-OCH₂CH(OC₂H₅)₂ + NaCl

BzO-PEG-OCH₂CH(OC₂H₅)₂ + H₂ (Pd,C) → → BzH+HO-PEG-OCH₂CH(OC₂H₅)₂

HO-PEG-OCH₂CH(OC₂H₅)₂ + C₆H₅-CH=CH-CE=CH-COCl + (CH₃CH₃)₃N → C₆H₅-CH=CH-CH=CH-CO₂-PEG-OCH₂CH(OC₂H₅)₂ + (CH₃CH₂)₃NH⁺Cl⁻

a) Preparation of BzO-PEG-O-CH₂CH(OC₂H₅)₂: In a 3-necked, 500 ml round-bottom flask were placed 300 ml of dioxane and 14 g of BzO-PEG-OH (MW=3400, 0.0040 moles). The resulting solution was then azeotropically dried by distillation under N₂ of 130 ml of solvent. After cooling the solution, finely powdered NaOH (0.8 g, 0.02 moles) and ClCH₂CH(OC₂H₅)₂ (3 ml, 0.02 moles) were added under N₂ and the resulting suspension was rapidly stirred while refluxing over a 24 h period. Thirty ml of dioxane was then removed by distillation and the rapidly stirred solution was refluxed under N₂ for an additional 24 h. The suspension was then cooled and filtered with the addition of Celite^{R}. The filtrate was evaporated under vacuum and 200 ml of ethyl ether was added to the residual oil. The resulting precipitate was collected by filtration and dried under vacuum at room temperature to obtain a tan powder (13.6 g). The powder was dissolved in CH₂Cl₂ (35 ml) and reprecipitated by the addition of 500 ml of cold ethyl ether. The precipitate was collected by filtration and dried under vacuum at room temperature to obtain 13.0 g of BzO-PEG-OCH₂CH(OC₂H₅)₂ as a white powder (purity 94-98% by ¹H nmr). ¹H nmr (DMSO-d₆): δ 1.11 (t, OCH₂CH₃); 3.5 1 (br m, O-CH₂CH₂-O), 4.48 (s, C₆H₅-CH₂O⁻); 4.55 (t. -CH(OC₂H₅)₂), 7.32 (s, C₆H₅-)
b) Preparation of HO-PEG-O CH₂CH(OC₂H₅)₂: BzO-PEG-OCH₂CH(OC₂H₅)₂ (13 g) was dissolved in 150 ml of 95% ethanol and 6.5 g of 10% Pd on charcoal was added under N₂. The suspension was shaken 70 h under H₂ (40 psi) and the suspension filtered. The residual catalyst was washed with 2x25 ml of boiling chloroform and the washings combined with the ethanol filtrate and evaporated under vacuum to obtain a clear, colorless oil. To the oil was added 400 ml of cold ethyl ether and the resulting precipitate collected by filtration to obtain, after vacuum drying at room temperature, 11.3 g of HO-PEG-OCH₂CH(OC₂H₅)₂ as a white powder (92% pure by ¹H nmr). ¹H nmr (DMSO-d₆): δ 1.10, (t, OCH₂CH₃), 3.51 (br m, O-CH₂CH₂-O), 4.55, (m, HO + -CH(OCH₂CH₃)₂)
c) Preparation of C₆H₅-CH=CH-CH=CH-CO₂PEG-OCH₂CH(OC₂H₅)₂: A solution of cinnamylideneacetic acid (1.7 g, 0.01 moles) and thionyl chloride (3 ml, 0.04 moles) in 50ml of hexane were refluxed under N₂ for 4 h, then filtered to remove a small amount of dark solid and the filtrate evaporated under vacuum. The residue was dried under vacuum overnight at room temperature to obtain 1.5 g of cinnamylideneacetyl chloride as a yellow solid, m.p. 51-52°C.)

A solution of HO-PEG-O CH₂CH(OC₂H₅)₂ (3.4 g, 1.0 mmole) in toluene (50 ml) was azeotropically distilled under nitrogen for 2 h to remove traces of water, then cooled to room temperature. Triethylamine was distilled from KOH under nitrogen and 0.28 ml (2 mmoles) of the fresh distillate was injected under nitrogen into the solution of HO-PEG-O CH₂CH(OC₂H₅)₂ in toluene. To the resulting solution was added cinnamylidene acetyl chloride (C₆H₅CH=CH-CH=CHCOCl) dropwise under nitrogen at room temperature with rapid stirring. Stirring was allowed to continue for three days under the same conditions and the white precipitate was removed by filtration. The filtrate was evaporated to 20 ml under vacuum and 300 ml of cold ether was added. The pale yellow precipitate was collected by filtration and dried under vacuum to obtain 3.4 g of pale yellow powder. The powder was dissolved in methylene chloride and extracted once with 50 ml of aqueous saturated sodium chloride and once with water. The organic phase was dried over sodium sulfate, evaporated to 25 ml, and 300 ml of cold ether was added with swirling. The resulting precipitate was collected by filtration and dried under vacuum at room temperature to obtain 3.05 g (86%) of C₆H₅-CH=CH-CH=CH-CO₂PEG-OCH₂CH(OC₂H₅)₂ as a pale yellow powder. ¹H nmr (DMSO-d₆): δ 1.11 ppm (t, CH₃CH₂O-, 3.51 ppm (m, PEG-O-CH₂CH₂-O + CH₃CH₂O-); 4.20ppm (t,-CH₂O₂C-), 4.52ppm (t, -CH(OC₂H₅)₂) 6.11 (d, =CH, 7.57-7.12 (comp. mult., C₆H₅- + =CH) Purity by nmr: 89-96%

### EXAMPLE9

### PREPARATION OF NHS-O₂CCH₂CH₂COO-PEG-OCH₂CH₂CH(OC₂H₅)₂ REACTIONS:

BzO-PEG-OH+MsCl+(H₅C₂)₃N → BzO-PEG-OMs+(H₅C₂)₃N⁺Cl⁻

BzO-PEG-OMs + NaH + HOCH₂CH₂CH(OC₂H₅)₂ → → BzO-PEG-OCH₂CH₂CH(OC₂H₅)₂

BzO-PEG-OCH₂CH₂CH(OC₂H₅)₂ + H₃ (Pd,C) → → BzH + HO-PEG-OCH₂CH₂CH(OC₂H₃)₂

HO-PEG-OCH₂CH₂CH(OC₂H₅)₂ + succinic anhydride + pyridine → → HO₂CCH₂CH₂CO₂-PEG-OCH₂CH₂CH(OC₂H₅)₂

HO₂CCH₂CH₂CO₂-PEG-OCH₂CH₂CH(OC₂H₅)₂ + DCC +NHS → → NH5-O₂CCH₂CH₂CO₂-PEG-OCH₂CH₂CH(OC₂H₅)₂

a) Preparation of BzO-PEO-OMs: BzO-PEG-OH (MW=3400, 25 g, 7.35 mmol) in 150 ml of toluene was azeotropically distilled for 1 hour under nitrogen and the solution was cooled to room temperature. To the solution was added 20 ml of dry methylene chloride, 1.14 ml of dry triethylamine (8.16 mmol) and 0.61 ml of dry mesyl chloride (7.86 mmol) dropwise. The solution was stirred at room temperature under nitrogen overnight and the reaction was quenched by adding 5 ml absolute ethanol. The mixture was concentrated under vacuum, tittered, concentrated again under vacuum and precipitated into ethyl ether. The product was collected by filtration and dried *_{in vacuo.}* Yield 23 g (100%). ¹H nmr (DMSO-d₆): δ 3.5 (br m, PEG), 4.31 (t, OCH₂CH₂OMs), 4.49 (s, C₆H₅-CH₂-O-PEG-), 7.33 (s + comp m., C₆H₅-CH₂-OPEG-).
b) Preparation of BzO-PEG-OCH₂CH₂CH(OC₂H₅)₂: A solution of 3,3-diethoxypropanol (9.806 g, 66.2 mmol) was azeotropically distilled in 90 ml of toluene for 1 hour under nitrogen. After cooling to room temperature the solution was added to a dispersion of sodium hydride (60% in mineral oil, 2.75 g, 68.7 mmol) in 50 ml of anhydrous toluene. The solution was mixed for 2 h under mild heating at 35°C and then filtered. The filtrate was added to an azeotropically distilled solution of BzOPEG-OMs (23 g, 6.76 mmol) in 150 ml toluene. The mixture was stirred for 20 hours at 125°C under a nitrogen atmosphere. The mixture was concentrated under vacuum and the residue was dissolved in 80 ml of methylene chloride. The solution was filtered and the product was precipitated with 1 liter of cold isopropyl alcohol. The product was collected by filtration and dried *in vacuo.* The powder was dissolved in 100 ml of deionized water and extracted three times with 200 ml methylene chloride. The mixture was concentrated under vacuum, filtered and precipitated into ethyl ether. The product was collected by filtration and dried *in vacuo.* Yield 19 g (100%). ¹H nmr (DMSO-d₆): δ 1.10 (t, -CH(OCH₂CH₃)₂, 1.73 (q, -OCH₂CH₂-CH), 3.5 (br m, PEG), 4.49 (s, C₆H₅-CH₂-OPEG-), 4.56 (m, -CH(OCH₂CH₃)₂), 7.33 (s + comp m, C₆H₅-CH₂-O-PEG-).
c) Preparation of HO-PEG-OCH₂CH₂CH(OC₂H₅)₂: BzO-PEG-OCH₂CH₂CH(OC₂H₅)₂ (10 g, 2.94 mmol) was dissolved in 100 ml of 96% ethanol and 5.0 g of 10% Pd on charcoal was added under nitrogen. The suspension was shaken 48 h under H₂ (40 psi) and the suspension filtered. The residual catalyst was washed with methylene chloride. The product in the combined filtrate of methylene chloride and ethanol was concentrated under vacuum and filtered. The viscous solution was precipitated into cold ethyl ether, and the product recovered by filtration and dried *in vacuo.* Yield 15 g. ¹H nmr (DMSO-d₆): δ 1.10 (t, -CH(OCH₂CH₃)₂, 1.72 (q, -OCH₂CH₂-CH). 3.5 (br m, PEG), 4.55 (m, -CH(OCH₂CH₃)₂)
d) Preparation of HO₂CCH₂CH₂CO₂-PEG-OCH₂CH₂CH(OCH₂CH₃)₂: HO-PEG-OCH₂CH₂CH(OC₂H₅)₂ (3 g, 0.88 mmol) and BHT (5 mg) were dissolved in 20 ml of anhydrous toluene and azeotropically distilled at 120°C for 1 hour under nitrogen. After cooling the solution to 75°C, pyridine (0.36 ml) and succinic anhydride (0.353 g) were added and stirred at 75°C for 24 hours. The solution was concentrated under vacuum, filtered and precipitated into cold ethyl ether. The precipitate was recovered by filtration and dried *in vacuo.* The powder was reconstituted in 50 ml deionized water and 1 M sodium hydroxide was added dropwise to maintain a constant pH of 7.2 for 1 hour. 1N HCl was quickly added dropwise to obtain a pH of 3.0 and immediately extracted, 3 times into 100 ml methylene chloride. The product in the organic phase was dried over sodium sulfate, concentrated under vacuum, precipitated into cold ethyl ether, recovered by filtration and dried *in vacuo.* Yield 2.0 g (88%). ¹H nmr (DMSO-d₆): δ 1.10 (t, - CH(OCH₂CH₃)₂, 1.72 (q, -OCH₂CH₂-CH), 3.5 (br m, PEG), 4.12 (t, -CO₂CH₂-), 4.55 (t, -CH(OCH₂CH)₂).
e) Preparation of NHS-O₂CCH₂CH₂CO₂-PEG-OCH₂CH₂CH(OCH₂CH₃)₂: HO₂CCH₂CH₂CO₂-PEG-OCH₂CH₂CH(OCH₂CH₃)₂ (2.0 g, 0.56 mmol) was dissolved in 20 ml anhydrous methylene chloride under a nitrogen atmosphere. N-hydroxysuccinimide (105 mg, 0.91 mmol) was first added to the solution and then dicyclohexylcarbodiimide (174 mg, 0.84 mmol) was added. The solution was stirred overnight at room temperature under nitrogen atmosphere. The product was concentrated under vacuum, filtered, precipitated into cold ethyl ether, recovered by filtration and dried *in vacuo.* Yield 1.5 g (99%). ¹H nmr (DMSO-d₆): δ 1.10 (t, -CH(OCH₂CH₃)₂, 1.72 (q, -OCH₂CH₂-CH), 2.80 (s, NHS), 3.5 (br m, PEG), 4.12 (t, -CO₂CH₂-), 4.55 (t, -CH(OCH₂CH₃)₂).

The invention has been described in particular exemplified embodiments. However, the foregoing description is not intended to limit the invention to the exemplified embodiments.

## Claims

1. A method for preparing a heterobifunctional polymeric derivative, comprising:
(i) providing a polymer of formula W-O-Poly-OH, where W is a group which is removable by catalytic hydrogenolysis or by acid-catalyzed hydrolysis, and Poly represents a polymer moiety selected from the group consisting of poly(alkylene oxides), poly(oxyethylated polyols), poly(olefinic alcohols), and poly(acryloylmorpholine);
(ii) modifying the -OH moiety to produce a first functional group, wherein said first functional group is selected from the group consisting of: mesylate, tosylate, tresylate,
- O-(CH₂)ₙ-CO₂R₃, wherein n=1-6 and R₃ is an alkyl group,
- NHR₄, wherein R₄ is H, alkyl or an amine protecting group,
- O-(CH₂)ₙ-CH(ZR₅)₂. wherein n is a number of 1-6, Z is O or S, and R₅ is H or an alkyl group,
- O-(CH₂)ₙ-CHO, where n is 1-6,
; and
(iii) converting said W to a second functional group, said second functional group being different from said first functional group

2. The method of Claim 1, wherein W has a formula of ArC(R₁)(R₂)- where Ar represents a moiety selected from the group consisting of phenyl, substituted phenyl, biphenyl, substituted biphenyl, polycyclic aryls, substituted polycyclic aryls, and heterocyclic aryls, where R₁ and R₂ are H, alkyl or Ar, which is defined above.

3. The method of Claim 1, wherein said second functional group is -OH.

4. A method for preparing a heterobifunctional polymeric derivative comprising:
(i) providing a polymer of the formula Ar-C(R₁)(R₂)O-Poly-OH,
where Ar is selected from the group consisting of phenyl, substituted phenyl, biphenyl, substituted biphenyl, polycyclic aryls, substituted polycyclic aryls, and heterocyclic aryls,
where R₁ and R₂ are H, alkyl or Ar, where Ar is as defined above, and
where Poly is a polymeric moiety selected from the group consisting of poly(alkylene oxides), poly(oxyethylated polyols), poly(olefinic alcohols), and poly(acryloylmorpholine);
(ii) chemically modifying the -OH group to produce a first functional group -O-(CH₂)ₙ-CH(ZR₅)₂, wherein n is a number of 1-6, Z is O or S, and R₅ is H or an alkyl group;
(iii) removing the Ar-C(R₁)(R₂)O- group to produce a new hydroxyl group;
(iv) converting the new hydroxyl group to a second functional group, said second functional group being different from said first functional group.

5. The method of Claim 1 or Claim 4, wherein Poly is poly(ethylene glycol) represented by -CH₂CH₂O(CH₂CH₂O)ₙ-CH₂CH₂-, where n' is from about 8 to about 4000.

6. The method of Claim 1 or 4, wherein Poly is a copolymer of ethylene oxide and propylene oxide.

7. The method of Claim 4, wherein said first functional group is - methoxy.

8. The method of claim 1 or 4, wherein said second functional group is selected from the group consisting of
mesylate; tosylate, tresylate,
-O-(CH₂)ₙ-CO₂H, wherein n=1-6,
-O-(CH₂)ₙ-CO₂R₃, wherein n=1-6 and R₃ is an alkyl group,
-NHR₄. wherein R₄ is H, alkyl or an amine protecting group,
-O-(CH₂)ₙ-CH(ZR₅)₂, wherein n is a number of 1-6, Z is O or S, and R₅ is H or an alkyl group, and
-O-(CH₂)ₙ-CHO, where n is 1-6.

9. The method of Claim 4, wherein said second functional group is - O-(CH₂)ₙ-CH(ZR₅)₂ where n is a number of 1-6, Z is O or S, and R₅ is H or an alkyl group.

10. The method of Claim 4, wherein said second functional group is - O-(CH₂)ₙ-CHO, where n is 1-6.

11. The method of Claim 1, wherein said first functional group is - O-(CH₂)ₙ-CH(ZR₅)₂, wherein n is a number of 1-6, Z is O or S, and R₅ is H or an alkyl group.

12. The method of claim 1 or claim 4, wherein the polymer in step (i) has the formula C₅H₆-CH₂-O-Poly-OH.

13. The method of claim 4, wherein said removing step (iii) comprises catalytic hydrogenolysis or acid-catalyzed hydrolysis.

14. The method of claim 4 for use in inhibiting the reactivity of HO-PEG-OH in a mixture, wherein:
Poly is polyethylene glycol (PEG),
Ar-C(R₁)(R₂)O- is C₅H₆-CH₂-O-,
step (ii) comprises chemically modifying the -OH group by alkylation to produce an -OR group, where R is alkyl, and
step (iii) comprises catalytic hydrogenation.

15. The method of claim 14, wherein the alkyl group is methyl.

16. A method for attaching a polymeric derivative to a drug or macromolecule, comprising the preparation of the polymeric derivative using the methods of claims 1, 4, or 14, and the additional step of attaching the polymeric derivative to said drug or macromolecule.

## Patentansprüche

1. Verfahren zur Herstellung eines heterobifuktionellen polymeren Derivats, bei dem man:
(i) ein Polymer der Formel W-O-Poly-OH bereitstellt, wobei W für eine Gruppe steht, die durch katalytische Hydrogenolyse oder durch säurekatalysierte Hydrolyse abspaltbar ist, und Poly für eine Polymergruppierung aus der Gruppe bestehend aus Poly(alkylenoxiden), Poly(oxyethylierten Polyolen), Poly(olefinischen Alkoholen) und Poly-(acryloylmorpholin) steht;
(ii) die -OH-Gruppierung modifiziert, wobei man eine erste funktionelle Gruppe erhält, die aus der Gruppe bestehend aus:
Mesylat, Tosylat, Tresylat,
-O-(CH₂₎ₙ-CO₂R₃, worin n = 1-6 und R₃ für eine Alkylgruppe steht,
-NHR₄, worin R₄ für H, Alkyl oder eine Amin-Schutzgruppe steht,
-O-(CH₂₎ₙ-CH(ZR₅)₂, worin n für eine Zahl von 1-6 steht, Z für O oder S steht und R₅ für H oder eine Alkylgruppe steht,
-O-(CH₂)ₙ-CHO, wobei n für 1-6 steht,
ausgewählt ist; und
(iii) das W in eine zweite funktionelle Gruppe umwandelt, welche von der ersten funktionellen Gruppe verschieden ist.

2. Verfahren nach Anspruch 1, bei dem W die Formel Ar-C(R₁)(R₂)- aufweist, wobei Ar für eine Gruppierung aus der Gruppe bestehend aus Phenyl, substituiertem Phenyl, Biphenyl, substituiertem Biphenyl, polycyclischen Arylresten, substituierten polycyclischen Arylresten und heterocyclischen Arylresten ausgewählt ist, wobei R₁ und R₂ für H, Alkyl oder Ar gemäß obiger Definition stehen.

3. Verfahren nach Anspruch 1, bei dem es sich bei der zweiten funktionellen Gruppe um -OH handelt.

4. Verfahren zur Herstellung eines heterobifuktionellen polymeren Derivats, bei dem man:
(i) ein Polymer der Formel Ar-C(R₁) (R₂)O-Poly-OH bereitstellt, wobei Ar aus der Gruppe bestehend aus Phenyl, substituiertem Phenyl, Biphenyl, substituiertem Biphenyl, polycyclischen Arylresten, substituierten polycyclischen Arylresten und heterocyclischen Arylresten ausgewählt ist, wobei R₁ und R₂ für H, Alkyl oder Ar gemäß obiger Definition stehen und
wobei Poly für eine polymere Gruppierung aus der Gruppe bestehend aus Poly(alkylenoxiden), Poly-(oxyethylierten Polyolen), Poly(olefinischen Alkoholen) und Poly(acryloylmorpholin) steht;
(ii) die -OH-Gruppierung chemisch modifiziert, wobei man eine erste funktionelle Gruppe -O-(CH₂)ₙ-CH(ZR₅)₂ erhält, worin n für eine Zahl von 1-6 steht, Z für O oder S steht und R₅ für H oder eine Alkylgruppe steht;
(iii) die Ar-C(R₁) (R₂)O-Gruppe abspaltet, wobei man eine neue Hydroxylgruppe erhält;
(iv) die neue Hydroxylgruppe in eine zweite funktionelle Gruppe umwandelt, welche von der ersten funktionellen Gruppe verschieden ist.

5. Verfahren nach Anspruch 1 oder Anspruch 4, bei dem Poly für Poly(ethylenglykol), das durch -CH₂CH₂O(CH₂CH₂O)ₙ'-CH₂CH₂- wiedergegeben wird, wobei n' etwa 8 bis etwa 4000 beträgt, steht.

6. Verfahren nach Anspruch 1 oder 4, bei dem Poly für ein Copolymer von Ethylenoxid und Propylenoxid steht.

7. Verfahren nach Anspruch 4, bei dem es sich bei der ersten funktionellen Gruppe um Methoxy handelt.

8. Verfahren nach Anspruch 1 oder 4, bei dem die zweite funktionelle Gruppe aus der Gruppe bestehend aus
Mesylat, Tosylat, Tresylat,
-O-(CH₂)ₙ-CO₂H, worin n = 1-6,
-O-(CH₂)ₙ-CO₂R₃, worin n = 1-6 und R₃ für eine Alkylgruppe steht,
-NHR₄, worin R₄ für H, Alkyl oder eine Amin-Schutzgruppe steht,
-O-(CH₂)ₙ-CH(ZR₅)₂, worin n für eine Zahl von 1-6 steht, Z für O oder S steht und R₅ für H oder eine Alkylgruppe steht, und
-O-(CH₂)ₙ-CHO, wobei n für 1-6 steht,
ausgewählt ist.

9. Verfahren nach Anspruch 4, bei dem es sich bei der zweiten funktionellen Gruppe um -O-(CH₂)ₙ-CH(ZR₅)₂, wobei n für eine Zahl von 1-6 steht, Z für O oder S steht und R₅ für H oder eine Alkylgruppe steht, handelt.

10. Verfahren nach Anspruch 4, bei dem es sich bei der zweiten funktionellen Gruppe um -O-(CH₂)ₙ-CHO, wobei n für 1-6 steht, handelt.

11. Verfahren nach Anspruch 1, bei dem es sich bei der ersten funktionellen Gruppe um -O-(CH₂)ₙ-CH(ZR₅)₂, wobei n für eine Zahl von 1-6 steht, Z für O oder S steht und R₅ für H oder eine Alkylgruppe steht, handelt.

12. Verfahren nach Anspruch 1 oder Anspruch 4, bei dem das Polymer in Schritt (i) die Formel C₅H₆-CH₂-O-Poly-OH aufweist.

13. Verfahren nach Anspruch 4, bei dem der Abspaltungsschritt (iii) eine katalytische Hydrogenolyse oder eine säurekatalysierte Hydrolyse umfasst.

14. Verfahren nach Anspruch 4 zur Verwendung bei der Inhibierung der Reaktivität von HO-PEG-OH in einer Mischung, bei dem:
Poly für Polyethylenglykol (PEG) steht,
Ar-C(R₁)(R₂)O- für C₅H₆-CH₂-O- steht,
Schritt (ii) die chemische Modifizierung der -OH-Gruppe durch Alkylierung zu einer -OR-Gruppe, wobei R für Alkyl steht, umfasst und
Schritt (iii) eine katalytische Hydrierung umfasst.

15. Verfahren nach Anspruch 14, bei dem es sich bei der Alkylgruppe um Methyl handelt.

16. Verfahren zum Verknüpfen eines polymeren Derivats mit einem Arzneistoff oder Makromolekül, bei dem man das polymere Derivat unter Verwendung der Verfahren der Ansprüche 1, 4 oder 14 herstellt und zusätzlich das polymere Derivat mit dem Arzneistoff oder Makromolekül verknüpft.

## Revendications

1. Méthode de préparation d'un dérivé polymère hétéro-bifonctionnel, comprenant :
(i) la fourniture d'un polymère de formule W-O-Poly-OH, où W est un groupement qui peut être éliminé par hydrogénolyse catalytique ou par hydrolyse catalysée à l'acide, et Poly représente un motif polymère choisi dans le groupe constitué par les poly(oxydes d'alkylène), les poly(polyols oxyéthylés), les poly(alcools oléfiniques) et la poly(acryloyl-morpholine) ;
(ii) la modification du motif -OH afin de produire un premier groupement fonctionnel, où ledit premier groupement fonctionnel est choisi dans le groupe constitué par :
mésylate, tosylate, trésylate,
-O-(CH₂)ₙ-CO₂R₃, où n=1-6 et R₃ est un groupement alkyle,
-NHR₄, où R₄ est H, alkyle ou un groupement protecteur d'amine,
-O-(CH₂)ₙ-CH(ZR₅)₂, où n est un nombre valant 1-6, Z est O ou S, et R₅ est H ou un groupement alkyle,
-O-(CH₂)ₙ-CHO, où n vaut 1-6 ; et
(iii) la conversion dudit W en un deuxième groupement fonctionnel, ledit deuxième groupement fonctionnel étant différent dudit premier groupement fonctionnel.

2. Méthode selon la revendication 1, dans laquelle W répond à la formule Ar-C(R₁)(R₂)- où Ar représente un motif choisi dans le groupe constitué par phényle, phényle substitué, biphényle, biphényle substitué, aryle polycyclique, aryle polycyclique substitué, et aryle hétérocyclique, où R₁ et R₂ sont H, alkyle ou Ar, qui est défini ci-dessus.

3. Méthode selon la revendication 1, dans laquelle ledit deuxième groupement fonctionnel est -OH.

4. Méthode de préparation d'un dérivé polymère hétéro-bifonctionnel, comprenant :
(i) la fourniture d'un polymère de formule ArC(R₁)(R₂)-O-Poly-OH,
où Ar est choisi dans le groupe constitué par phényle, phényle substitué, biphényle, biphényle substitué, aryle polycyclique, aryle polycyclique substitué, et aryle hétérocyclique,
où R₁ et R₂ sont H, alkyle ou Ar, où Ar est tel que défini ci-dessus, et
où Poly représente un motif polymère choisi dans le groupe constitué par les poly(oxydes d'alkylène), les poly(polyols oxyéthylés), les poly(alcools oléfiniques) et la poly(acryloylmorpholine) ;
(ii) la modification chimique du motif -OH afin de produire un premier groupement fonctionnel, -O-(CH₂)ₙ-CH(ZR₅)₂, où n est un nombre valant 1-6, Z est O ou S, et R₅ est H ou un groupement alkyle ;
(iii) l'élimination du groupement Ar-C(R₁)(R₂)O- afin de produire un nouveau groupement hydroxyle ;
(iv) la conversion du nouveau groupement hydroxyle en un deuxième groupement fonctionnel, ledit deuxième groupement fonctionnel étant différent dudit premier groupement fonctionnel.

5. Méthode selon la revendication 1 ou la revendication 4, dans laquelle Poly est poly(éthylène glycol) représenté par -CH₂CH₂O(CH₂CH₂O)_{n'}-CH₂CH₂-, où n' va d'environ 8 à environ 4000.

6. Méthode selon la revendication 1 ou 4, dans laquelle Poly est un copolymère d'oxyde d'éthylène et d'oxyde de propylène.

7. Méthode selon la revendication 4, dans laquelle ledit premier groupement fonctionnel est -méthoxy.

8. Méthode selon la revendication 1 ou 4, dans laquelle ledit deuxième groupement fonctionnel est choisi dans le groupe constitué par :
mésylate, tosylate, trésylate,
-O-(CH₂)ₙ-CO₂H, où n=1-6,
-O-(CH₂)ₙ-CO₂R₃, où n=1-6 et R₃ est un groupement alkyle,
-NHR₄, où R₄ est H, alkyle ou un groupement protecteur d'amine,
-O-(CH₂)ₙ-CH(ZR₅)₂, où n est un nombre valant 1-6, Z est O ou S, et R₅ est H ou un groupement alkyle, et
-O-(CH₂)ₙ-CHO, où n vaut 1-6.

9. Méthode selon la revendication 4, dans laquelle ledit deuxième groupement fonctionnel est -O-(CH₂)ₙ-CH(ZR₅)₂, où n est un nombre valant 1-6, Z est O ou S, et R₅ est H ou un groupement alkyle.

10. Méthode selon la revendication 4, dans laquelle ledit deuxième groupement fonctionnel est -O-(CH₂)ₙ-CHO, où n vaut 1-6.

11. Méthode selon la revendication 1, dans laquelle ledit premier groupement fonctionnel est -O-(CH₂)ₙ-CH(ZR₅)₂, où n est un nombre valant 1-6, Z est O ou S, et R₅ est H ou un groupement alkyle.

12. Méthode selon la revendication 1 ou la revendication 4, dans laquelle le polymère à l'étape (i) répond à la formule C₅H₆-CH₂-O-Poly-OH.

13. Méthode selon la revendication 4, dans laquelle ladite étape d'élimination (iii) comprend une hydrogénolyse catalytique ou une hydrolyse catalysée à l'acide.

14. Méthode selon la revendication 4, pour une utilisation dans l'inhibition de la réactivité de HO-PEG-OH dans un mélange, dans laquelle ;
Poly est le polyéthylène glycol (PEG),
Ar-C(R₁)(R₂)O- est C₅H₆-CH₂-O-,
l'étape (ii) comprend la modification chimique du groupement -OH par alkylation afin de produire un groupement -OR, où R est alkyle, et
l'étape (iii) comprend une hydrogénation catalytique.

15. Méthode selon la revendication 14, dans laquelle le groupement alkyle est méthyle.

16. Méthode destiné à fixer un dérivé polymère à un médicament ou une macromolécule, comprenant la préparation du dérivé polymère en utilisant les méthodes des revendications 1, 4 ou 14, et l'étape supplémentaire consistant à fixer le dérivé polymère audit médicament ou à ladite macromolécule.
